**Europäisches Patentamt**

**European Patent Office**

**Office européen des brevets**

(19)

(11) Publication number: **0 097 189**
**B1**

# EUROPEAN PATENT SPECIFICATION

(12)

(45) Date of publication of patent specification: **25.03.87**

(21) Application number: **83900026.2**

(22) Date of filing: **10.12.82**

(86) International application number:
**PCT/EP82/00259**

(87) International publication number:
**WO 83/02187 23.06.83 Gazette 83/15**

(51) Int. Cl.⁴: **G 08 B 17/10,** G 08 B 29/00

(54) **LIGHT EXTINCTION SMOKE DETECTOR.**

(30) Priority: **11.12.81 JP 199721/81**

(43) Date of publication of application:
**04.01.84 Bulletin 84/01**

(45) Publication of the grant of the patent:
**25.03.87 Bulletin 87/13**

(84) Designated Contracting States:
**CH DE FR LI**

(56) References cited:
**GB-A-2 059 128**
**GB-A-2 076 534**
**US-A-4 185 278**
**US-A-4 203 100**

**Patent Abstracts of Japan, volume 2, no. 55, 21
April 1978, page 1196E78**

(73) Proprietor: **Nohmi Bosai Kogyo Co., Ltd.
No. 7-3, Kudan Minami 4-chome
Chiyoda-ku Tokyo 102 (JP)**

(72) Inventor: **YASUKAWA, Makoto
Nohmi Bosai Kogyo CO. Ltd. No. 7-3 Kudan-
Minami
4-chome Chiyoda-ku Tokyo (JP)**
Inventor: **ISHII, Kenji
Nohmi Bosai Kogyo CO. Ltd. No. 7-3 Kudan-
Minami
4-chome Chiyoda-ku Tokyo (JP)**

(74) Representative: **Tiemann, Ulrich, Dr.-Ing.
c/o Cerberus AG Patentabteilung Alte
Landstrasse 411
CH-8708 Männedorf (CH)**

Courier Press, Leamington Spa, England.

## Description

Technical field
The invention relates to a light extinction smoke detector comprising a light source emitting pulse light and a light receiver disposed outside the path of light from the source so as to receive radiation scattered from smoke particles in the path of the light. Such smoke detectors are used e.g. as fire alarms.

Background art
In general the light extinction type smoke detector produces a fire signal upon detecting the extent to which the output of a photoelectric element receiving light from a light source that is spaced across the passageway for smoke, is reduced by the smoke of a fire. Therefore, it has the disadvantage that the interception of a light path extending from the light source to the photoelectric element, attributed to the shift of the light path or the like, might reduce the output of the photoelectric element, to provide a false alarm. The known means for eliminating this disadvantage is as stated below.

On the output side of the photoelectric element which receives the light from the light source that is spaced across a smoke passageway, there are connected a first level discriminator which detects the reduction in the output level of the photoelectric element ascribable to the smoke of the fire, and a second level discriminator which detects a rapid reduction in the output level of the photoelectric element ascribable to the interception of the light path extending from the light source to the photoelectric element or which detects the rate of variation of the output level. The output of the first level discriminator enters one of the input terminals of a logical product circuit through a delay circuit, and the fire signal is provided from the output terminal of the said logical product circuit. The output of the second level discriminator enters the set input terminal of an R/S flip-flop circuit, the set output of which enters the other input terminal of the said logical product circuit through a "not" circuit and is also used as a light path interception signal.

Such means has no problem in a case where the received light output of the photoelectric element decreases slowly due to the smoke of the fire but does not fall below the second level. However, in a case where high density smoke first enters the light path, the output of the photoelectric element becomes lower than the second level and subsequently when low density smoke enters rendering the output of the photoelectric element to become a value somewhere between the first and second levels, the output of the second level discriminator enters the input terminal of the logical product circuit through both the R/S flip-flop circuit and the "not" circuit, to inhibit the logical product circuit in providing the fire signal and simultaneously provides the light path interception signal. In addition, when low density smoke and high smoke density enter the light path alternately, the fire signal is provided if the output of the photoelectric element is above the second level. However, when the above output becomes lower than the second level, the second level discriminator produces an output, which inhibits the continuous provision of the fire signal and provides the light path interception signal. Furthermore, in this case, it is inevitable that the light path interception signal is produced without the provision of the fire signal, if the delay time of the delay circuit connected to the first level discriminator is longer than the period of time during which the smoke density changes.

A light extinction smoke sensor as defined in the preamble of claim 1 is known in GB—A—2 059 128 which is adapted to eliminate the drawbacks caused by both aging of the light emitter and receiver and depositing of dust in the optical system.

Disclosure of invention
The invention aims at obtaining a smoke detector which uses a simple pulse light or modulated pulse light as light source instead of continuous light to save energy consumed, and which is capable of avoiding a false alarm by correctly discriminating and detecting smoke generated by fire and interception of the light path extending from the light source to the photoelectric element to receive the light.

These aspects of the invention are achieved by the features of claim 1 and 2.

The accompanying drawings, which are incorporated in and constitute a part of this specification, illustrate two embodiments of the present invention and, together with the specification, serve to explain the principles of the invention.

Brief description of the drawings
Figure 1 is a circuit diagram of a first embodiment of the invention.

Figures 2 and 3 are time charts showing the operating states of various portions of the embodiment shown in Figure 1.

Figures 4 and 5 are time charts showing the operating states of various parts in the cases where the interception of the light path has occurred.

Figure 6 is a circuit diagram of the essential portions of a second embodiment of the invention.

Figures 7 and 8 are time charts corresponding to Figures 2 and 4 and showing the operating states of various parts of the embodiment shown in Figure 6.

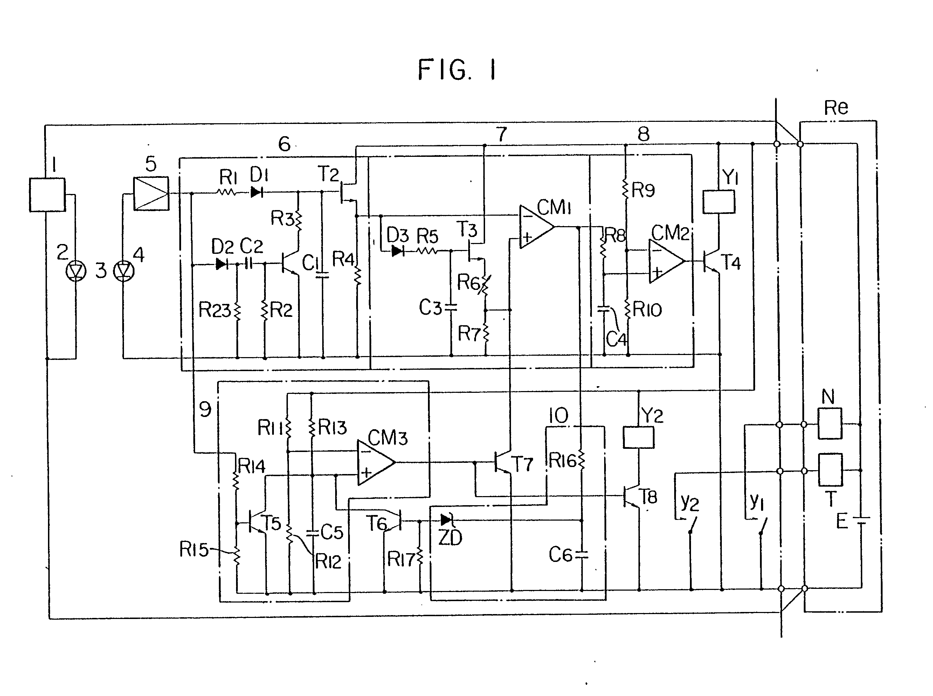

Modes for carrying out the invention
Figure 1 is a circuit diagram of a first embodiment of the invention. Numeral 1 designates a generator for a simple pulse voltage, numeral 2 a light emitting element, such as a light emitting diode, which emits pulse light with the output of the generator 1, numeral 3 a space through which smoke from a fire passes, numeral 4 a photoelectric element, such as a solar cell, which receives the pulse light emitted by the element 2 across the space 3 intervening therebetween, and numeral 5 an amplifier for the pulse voltage developed in element 4. Numeral 6 indicates a circuit for holding the peak value of the pulse voltage, constructed of a circuit which charges a capacitor $C_1$ with the output voltage of the amplifier 5 through a resistor $R_1$ as well as through diode $D_1$, and a circuit which differentiates the output voltage of the amplifier 5 through a series circuit consisting of a diode $D_2$, a capacitor $C_2$ and a resistor $R_2$ and turns "on" a transistor $T_1$ owing to a voltage drop having developed across the resistor $R_2$, so as to discharge the charge of the capacitor $C_1$ through a resistor $R_3$ as well as the transistor $T_1$, and which includes a discharging resistor $R_{23}$ for the capacitor $C_2$; and a source follower of a field-effect transistor $T_2$ whose conduction is controlled by the voltage of the capacitor $C_1$ and which includes an output resistor $R_4$. Numeral 7 indicates a fire discriminating circuit which provides a fire signal when the source voltage of the field-effect transistor $T_2$ of the peak value holding circuit 6 has reached a predetermined proportion of a reference voltage. It includes a comparator $CM_1$ which has an inverting input terminal (−) supplied with the source voltage of the transistor $T_2$, and which has a noninverting input terminal (+) supplied with the reference voltage appearing at the intermediate point of the output resistor $R_6$ and $R_7$ of a source follower of a field-effect transistor $T_3$, the conduction of which is controlled by the voltage of a capacitor $C_3$ that is charged with the source voltage of the transistor $T_2$ through a diode $D_3$ as well as a resistor $R_5$. An output from the comparator $CM_1$ turns "on" transistor $T_4$ with the delay of a predetermined time $\tau_1$ through a delay circuit 8 for preventing any erroneous operation due to noise. The delay circuit 8 is constructed of a capacitor $C_4$ which is charged with the aforementioned output through a resistor $R_8$, and a comparator $CM_2$ which has a noninverting input terminal (+) supplied with the voltage of the capacitor $C_4$ and which has an inverting input terminal (−) supplied with a reference voltage at the intermediate point of resistor $R_9$ and $R_{10}$ that are connected to a power source E in a receiver Re in series with each other. Thus, the delayed output energizes a relay $Y_1$ so as to close its contact $y_1$, whereupon the fire signal is sent to a zone relay N in the receiver Re so as to operate it. On the other hand, numeral 9 indicates a light path interception-discriminating circuit. It includes a comparator $CM_3$ whose inverting input terminal (−) is supplied as a reference voltage with the voltage of the intermediate point of resistors $R_{11}$ and $R_{12}$ that are connected to the power source E in the receiver Re in series with each other, and whose noninverting input terminal (+) is supplied with the voltage of a capacitor $C_5$ that is charged through a resistor $R_{13}$ by the power source E; and a transistor $T_5$ for discharging the capacitor $C_5$, which is connected in parallel with the capacitor $C_5$ and which is

turned "on" through resistors $R_{14}$ and $R_{15}$ by the output voltage of the amplifier 5. Normally, the transistor $T_5$ is conductive, so that the voltage of the capacitor $C_5$ is null. However, when the light path has been intercepted to drastically lower the output of the amplifier 5 and to render the transistor $T_5$ nonconductive, the capacitor $C_5$ is charged. When, after lapse of a predetermined time $\tau_2$, the voltage of the capacitor $C_5$ has exceeded the reference voltage of the comparator $CM_3$, this comparator $CM_3$ produces an output. When the comparator $CM_3$ produces the output, a transistor $T_7$ turns "on" to make null the voltage of the noninverting input terminal (+) of the comparator $CM_1$ in the fire discriminating circuit 7, and the operation of the circuit 7 is inhibited. At the same time, a transistor $T_8$ turns "on", and a relay $Y_2$ is energized to close its contact $y_2$ and to send the light path interception signal, which operates a light path interception-indicating relay T in the receiver Re. Conversely, when the comparator $CM_1$ has produced its output, a transistor $T_6$ which has a resistor $R_{17}$ connected across the base and emitter thereof is turned "on" after lapse of a predetermined time $\tau_3$ through a delay circuit 10, which is constructed of a capacitor $C_6$ that is charged with the output voltage of the comparator $CM_1$ through a resistor $R_{16}$ and a Zener diode ZD that is turned "on" by the voltage of the capacitor $C_6$. Thus, the voltage of the noninverting input terminal (+) of the comparator $CM_3$ is made null, and the operation of the light path interception-discriminating circuit 9 is inhibited.

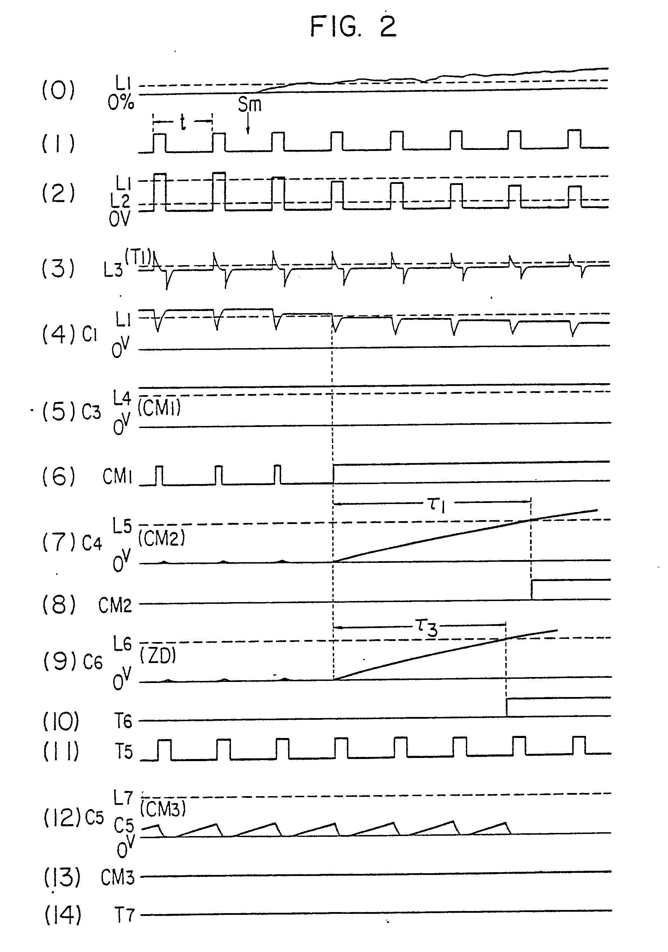

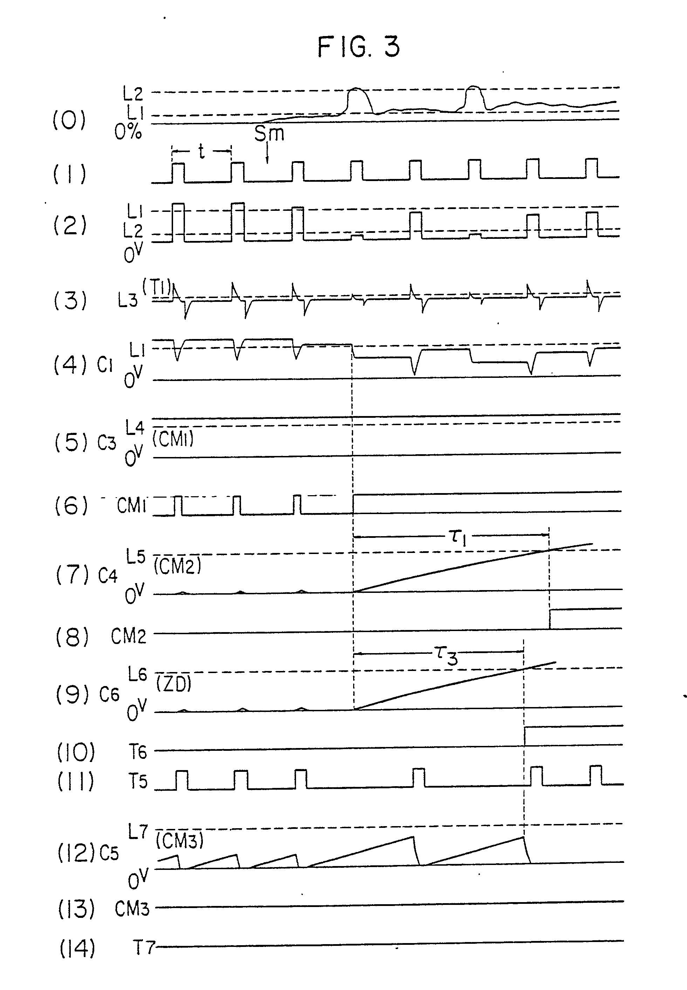

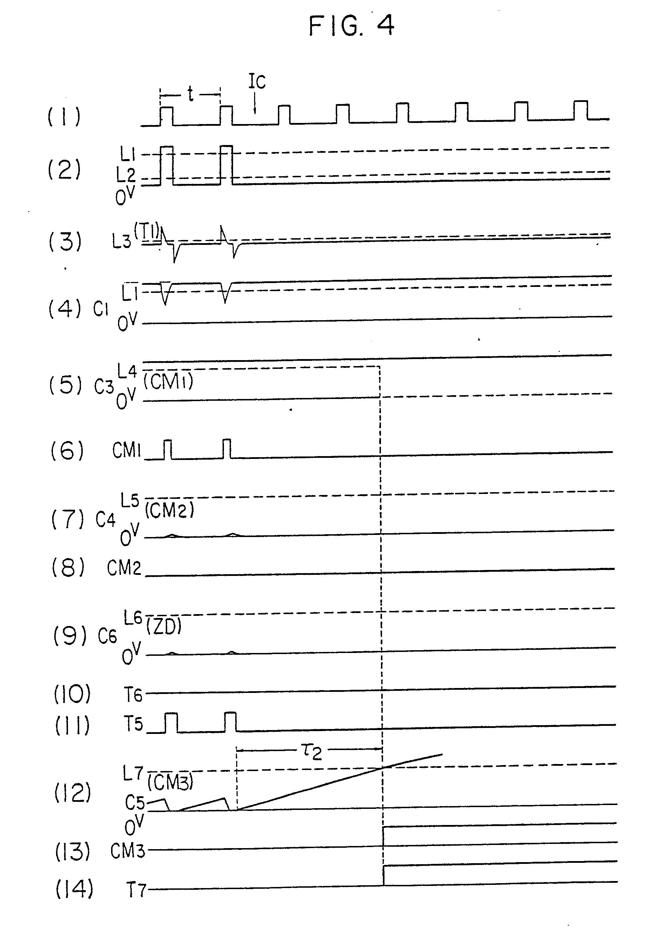

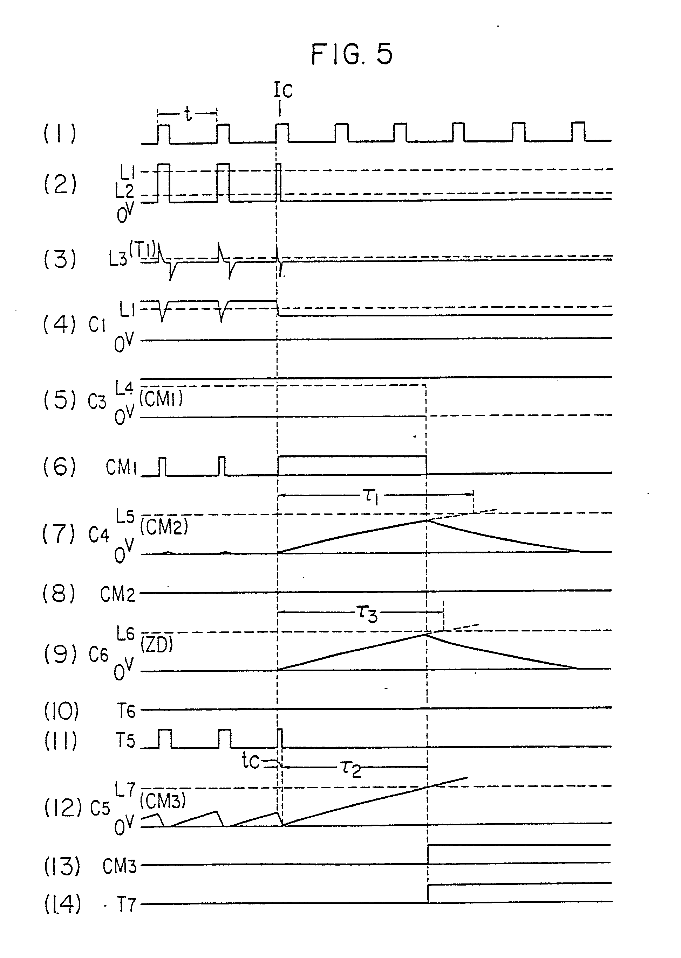

Figures 2 and 3 are time charts showing the operating states of various parts in the cases where smoke has entered the light path at points of time indicated by arrows Sm in the normal states as shown at (0), respectively. In the former case, the density of the smoke has changed slowly as indicated by a solid line, while in the latter case, the high density smoke equal to or above a light path interception level has entered initially, followed by the low density smoke equal to or above a fire level. Figures 4 and 5 are time charts showing the operating states of various parts in the cases where the interception of the light path has occurred at points of time indicated by arrows Ic in the normal states, respectively. In the time charts of Figures 2 to 5, (0) illustrates the density variation of the smoke in the light path, (1) the pulse light emission output of the light emitting element 2 having a period $t$, (2) the output of the amplifier 5 obtained by amplifying the light reception output voltage of the photoelectric element 4, (3) the voltage obtained by differentiating the output voltage of the amplifier 5, the illustrated voltage appearing across the resistor $R_2$ of the peak value holding circuit 6, (4) the peak voltage held in the capacitor $C_1$, (5) the maximum value of the output voltage of the amplifier 5; the illustrated value being stored in the capacitor $C_3$ of the fire discriminating circuit 7, (6) the output of the comparator $CM_1$, (7) the voltage of the capacitor $C_4$ of the delay circuit 8, (8) the output of

the comparator $CM_2$, (9) the voltage of the capacitor $C_6$ of the delay circuit 10, (10) the operating state of the transistor $T_6$, (11) the operating state of the transistor $T_5$ of the light path interception-discriminating circuit 9, (12) the voltage of the capacitor $C_5$, (13) the output of the comparator $CM_3$, and (14) the operating state of the transistor $T_7$. Symbol $L_1$ at (0), (2) and (4) in Figures 2 to 5 denotes the level which is to be determined the fire, symbol $L_2$ at (0) and (2) denotes the level which is to be determined the light path interception, symbol $L_3$ at (3) denotes that level of the base-emitter voltage of the transistor $T_1$ which is necessary for the conduction of this transistor, symbol $L_4$ at (5) denotes the level of the reference voltage which is applied to the non-inverting input terminal (+) of the comparator $CM_1$ and which corresponds to the fire level $L_1$ at (2) and (4), symbol $L_5$ at (7) denotes the level of the reference voltage which is applied to the inverting input terminal (−) of the comparator $CM_2$, symbol $L_6$ at (9) denotes the level of the Zener voltage of the Zener diode ZD, and symbol $L_7$ at (12) denotes the level of the reference voltage which is applied to the inverting input terminal (−) of the comparator $CM_3$.

Referring to Figures 2 to 5, while there is not any smoke in the smoke passage 3 and the light path is not intercepted, either, the differentiated voltage of the output voltage of the amplifier 5 as shown at (3) appears across the resistor $R_2$ each time the amplifier output voltage shown at (2) develops, so that the transistor $T_1$ turns "on" to discharge the charges of the capacitor $C_1$ through the resistor $R_3$ as shown at (4). However, when the above differentiated voltage comes below the level $L_3$, the transistor $T_1$ turns "off", so that the capacitor $C_1$ is recharged with the output voltage of the amplifier 5 and holds the peak value thereof. The held voltage is applied to the inverting input terminal (−) of the comparator $CM_1$ of the fire discriminating circuit 7. This voltage held by the capacitor $C_1$ becomes lower than the fire level $L_1$, as accordingly does the level $L_4$ of the reference voltage of the noninverting input terminal (+) of the comparator $CM_1$ of the fire discriminating circuit 7 as shown at (5), for a moment when the transistor $T_1$ has turned "on". Therefore, the comparator $CM_1$ produces its output as shown at (6), and the capacitors $C_4$ and $C_6$ are charged with the output of the comparator $CM_1$ as shown at (7) and (9) respectively. However, since, the output of the comparator $CM_1$ ceases immediately, the voltages of the capacitors $C_4$ and $C_6$ do not reach the level $L_5$ of the reference voltage of the comparator $CM_2$ and level $L_6$ of the Zener voltage of the Zener diode ZD respectively, so that the charges of these capacitors $C_4$ and $C_6$ are discharged through the comparator $CM_1$. On the other hand, the capacitor $C_5$ of the light path interception-discriminating circuit 9 is charged through the resistor $R_{13}$. However, each time the output of the amplifier 5 exceeds the light path interception level $L_2$ as shown at (2), the transistor $T_5$ turns "on" as

shown at (11), and the charges of the capacitor $C_5$ are discharged through the transistor $T_5$ as shown at (12), so that the voltage of the capacitor $C_5$ does not reach the level $L_7$ of the reference voltage applied to the inverting input terminal (−) of the comparator $CM_3$.

When, as illustrated at (0) in Figure 2, the smoke has started entering the light path at the point of time indicated by the arrow Sm and the density of the smoke slowly changes as indicated by the solid line, the output voltage of the amplifier 5 falls gradually with increase in the density of the smoke as shown at (2), and the differentiated voltage thereof shown at (3) falls gradually accordingly. Each time the differentiated voltage exceeds the level $L_3$, the transistor $T_1$ turns "on" to discharge the charges of the capacitor $C_1$ as shown at (4), and the discharged capacitor $C_1$ is recharged up to the peak value of the lowered output voltage of the amplifier 5, but as the output voltage of the amplifier 5 has become lower than the level $L_1$ at (2), the voltage held by the capacitor $C_1$ becomes lower than the level $L_1$, and the input of the inverting input terminal (−) of the comparator $CM_1$ becomes lower than the reference voltage $L_4$ of the noninverting input terminal (+), so that the comparator $CM_1$ continues to provide its output as shown at (6). As a result, the capacitors $C_4$ and $C_6$ are respectively charged with the output of the comparator $CM_1$ through the resistors $R_8$ and $R_{16}$ as shown at (7) and (9). On the other hand, the capacitor $C_5$ is charged through the resistor $R_{13}$ as shown at (12), just as in a normal state. However, since the output of the amplifier 5 is greater than the light path interception level $L_2$ as shown at (2), the transistor $T_5$ turns "on" as shown at (11) and discharges the charge of the capacitor $C_5$ each time the amplifier 5 provides its output. As shown at (9), the voltage of the capacitor $C_6$ increases and exceeds the level $L_6$ indicative of the Zener voltage of the Zener diode ZD after lapse of the predetermined time $\tau_3$. Then, the transistor $T_6$ turns "on" as shown at (10) and inhibits the comparator $CM_3$ to provide the output. Subsequently, the voltage of the capacitor $C_4$ increases as shown at (7) and exceeds the level $L_5$ of the reference voltage of the inverting input terminal (−) of the comparator $CM_2$ after a lapse of the predetermined time $\tau_1$. Then, the comparator $CM_2$ provides its output as shown at (8), the transistor $T_4$ is turned "on", and the relay $Y_1$ operates to close its contact $y_1$, so that the fire signal is provided.

Next, when as illustrated at (0) in Figure 3, the smoke has started entering the light path at the point of time indicated by the arrow Sm and high density smoke equal to or above the light path interception level $L_2$ has entered at first as indicated by the solid line, the output of the amplifier 5 immediately falls below the light path interception level $L_2$ as shown at (2), and the transistor $T_5$ of the light path interception-discriminating circuit 9 does not turn "on" as shown at (11), so that the capacitor $C_5$ continues to be charged as shown at (12). However, when low density smoke in between the fire level $L_1$ and the light path interception level $L_2$ subsequently enters, the output of the amplifier 5 becomes a value somewhere between the levels $L_1$ and $L_2$ as shown at (2), and the transistor $T_5$ turns "on" again as shown at (11), so that the charge on capacitor $C_5$ discharges as shown at (12). Therefore, in a case where the output of the amplifier 5 has fallen below the light path interception level $L_2$ for a short time, the comparator $CM_3$ of the light path interception-discriminating circuit 9 does not provide its output as illustrated at (13). As shown at (7) and (9), the respective capacitors $C_4$ and $C_6$ of the delay circuits 8 and 10 start being charged with the output of the comparator $CM_1$ of the fire discriminating circuit 7 illustrated at (6), at the time of which the output of the amplifier 5 has fallen below the fire level $L_1$ as shown at (2). When, as illustrated at (9), the voltage of the capacitor $C_6$ has reached the level $L_6$ of the Zener voltage of the Zener diode ZD after a lapse of the predetermined time $\tau_3$, the transistor $T_6$ turns "on" as shown at (10), to inhibit the operation of the light path interception-discriminating circuit 9. When, as illustrated at (7), the voltage of the capacitor $C_4$ has exceeded the level $L_5$ of the reference voltage applied to the inverting input terminal (−) of the comparator $CM_2$ of the delay circuit 8, after a lapse of the predetermined time $\tau_1$, the comparator $CM_2$ provides its output as shown at (8), the transistor $T_4$ turns 2 "on", and the relay $Y_1$ operates to close its contact $y_1$, so that the fire signal is provided.

As can be seen from such operating states, in both cases where smoke of a higher density than the light path interception level has entered the light path at first and then smoke of a density somewhere between the fire level $L_1$ and the light path interception level $L_2$ has entered, and where smoke of such high and low densities has entered alternately, the capacitor $C_5$ of the light path interception-discriminating circuit 9 has its charge discharged as shown at (12), when thin smoke somewhere between the levels $L_1$ and $L_2$ has entered. Therefore, the comparator $CM_3$ does not provide its output.

Figure 4 illustrates the operating states of the various parts in the case where the light path has been intercepted in the quiescent period of the light emission of the light emitting element 2. The arrow lc denotes the point of time at which the interception has occurred. Notwithstanding that the light emitting element 2 is continuing the pulse light emission as shown at (1), the amplifier 5 produces no output as shown at (2) from the point of time indicated by the arrow lc, because the photoelectric element 4 receives no light. As a result, the differentiated voltage of the amplifier output does not appear across the resistor $R_2$, and the capacitor $C_1$ continues to hold the peak voltage of the last output of the amplifier 5 as shown at (4). Since the comparator $CM_1$ does not produce its output as shown at (6) either, the capacitors $C_4$ and $C_6$ are no longer charged as shown at (7) and (9), respectively. On the other

hand, the capacitor $C_5$ of the light path interception-discriminating circuit 9 is charged through the resistor $R_{13}$ as shown at (12). Since the transistor $T_5$ has turned "off" due to the null output of the amplifier 5, the capacitor $C_5$ continues to be charged. After a lapse of the period of time $\tau_2$, the charged voltage of the capacitor $C_5$ exceeds the level $L_7$ of the reference voltage of the inverting input terminal (−) of the comparator $CM_3$, and this comparator $CM_3$ provides its output as shown at (13). Therefore, the transistor $T_7$ turns "on" as shown at (14) and makes null the level $L_4$ of the reference voltage of the noninverting input terminal (+) of the comparator $CM_1$ as shown at (5), so as to inhibit the comparator $CM_1$ from providing its output. Simultaneously, the transistor $T_8$ turns "on" to energize the relay $Y_2$ so as to close the contact $y_2$ thereof and to provide the light path interception signal.

Figure 5 illustrates the operating states of the various parts in the case where the light path has been intercepted at the point of time indicated by the arrow lc, during the light emission of the light emitting element 2. At this time, the period of time during which the photoelectric element receives the light is short on account of the interception of the light path. The width of the output voltage of the amplifier 5 narrows as shown at (2), the differentiated voltage of the amplifier output appearing across the resistor $R_2$ is sharp, and the conduction period of time of the transistor $T_1$ is short as compared with that in the normal state. In the short period, the charges of the capacitor $C_1$ are discharged, and the charged voltage thereof falls below the fire level $L_1$ of the comparator $CM_1$ as shown at (4). The capacitor $C_1$, after the discharge can scarcely be charged with such a narrow pulse width voltage as that of the amplifier 5. Since the lowered voltage held by the capacitor $C_1$ has become below the level $L_4$ of the reference voltage of the noninverting input terminal (+) of the comparator $CM_1$ corresponding to the fire level $L_1$, the comparator $CM_1$ produces its output as shown at (6), and the capacitors $C_4$ and $C_6$ are charged with this output as shown at (7) and (9) respectively. On the other hand, since the pulse width of the output voltage of the amplifier 5 is so narrow that the transistor $T_5$ turns on for a short time as shown at (11), whereby the charge of the capacitor $C_5$ is discharged as shown at (12). Immediately after completion of the discharge, the capacitor $C_5$ begins to be charged. When after a lapse of the period of time $\tau_2$, the voltage of the capacitor $C_5$ has exceeded the level $L_7$ of the reference voltage applied to the inverting input terminal (−) of the comparator $CM_3$, this comparator $CM_3$ produces its output as shown at (14). Then, the level $L_4$ of the reference voltage of the noninverting input terminal (+) of the comparator $CM_1$ becomes null as shown at (5), and the output of the comparator $CM_1$ becomes null as shown at (6), so that the charges of the capacitors $C_4$ and $C_6$ are discharged through the comparator $CM_1$ as shown at (7) and (9) respectively. At the same time, the output of the

comparator $CM_3$ turns "on" the transistor $T_8$, which energizes the relay $Y_2$ to close the contact $y_2$, so that the light path interception signal is provided. The relationships of the magnitudes of the periods of time $\tau_1$, $\tau_2$ and $\tau_3$ may be selected as the following inequalities where $t_c$ denotes the conduction time of the transistor $T_5$ corresponding to the pulse width of the pulse output shown at (2) and obtained by amplifying the received light output of the photoelectric element 4:

$$\tau_1 - t_c > \tau_2, \; \tau_3 - t_c > \tau_2, \; \tau_3 \leqq \tau_1$$

The reasons therefor are as stated below. When the interception of the light path has occurred during the light emission of the light emitting element 2 as illustrated at (1) in Figure 5, the comparator $CM_1$ of the fire discriminating circuit 7 produces its output continuously from the point of time of the initiation of the light emission as seen from (6), and the capacitors $C_4$ and $C_6$ begin to be charged as shown at (7) and (9) respectively. In addition, as seen from (12), the capacitor $C_5$ having begun to be charged since the point of time of the light path interception, this point of time being later than the point of time of the initiation of the charging of the capacitors $C_4$ and $C_6$ equal to the conduction time $t_c$ of the transistor $T_5$, continues to be charged as it is. Therefore $\tau_2$ must be shorter than the period of time obtained by subtracting $t_c$ from $\tau_1$ or $\tau_3$, in order that the light path interception signal may be provided before the generation of the fire signal and the inhibition of the operation of the light path interception-discriminating circuit. Moreover, the light path interception signal must not be provided before the fire signal is produced. Therefore, $\tau_3$ must not be longer than $\tau_1$.

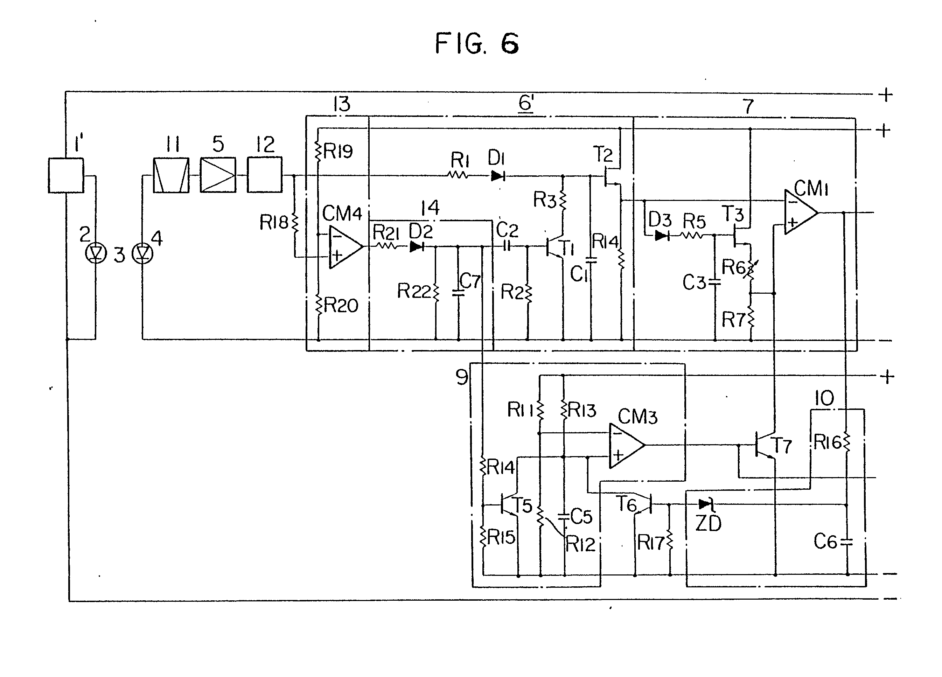

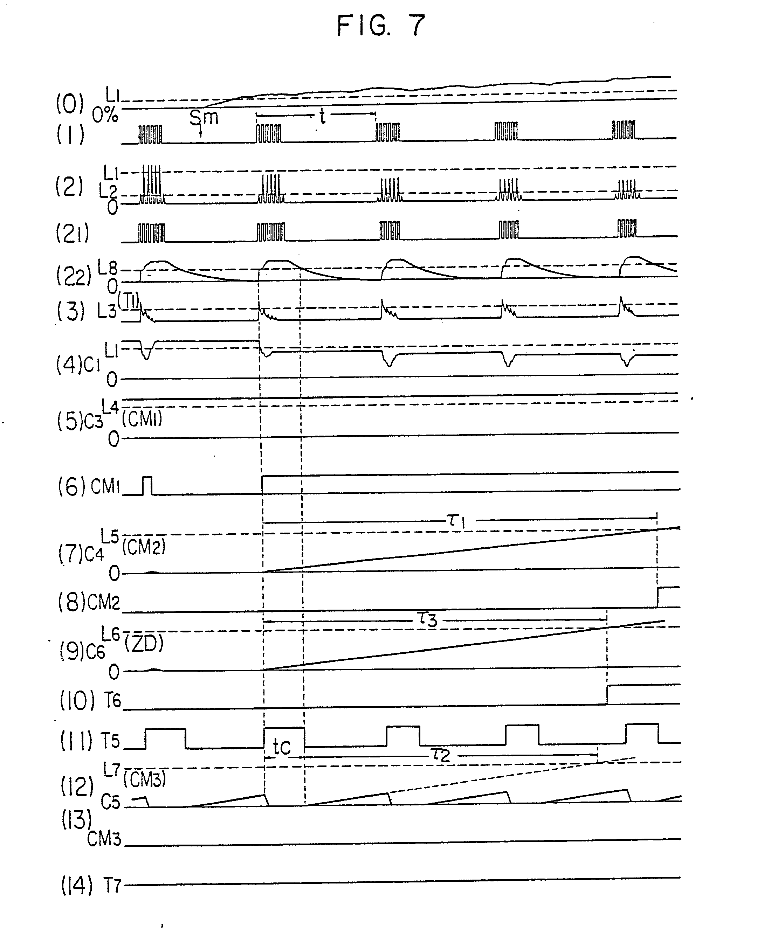

Figure 6 is a circuit diagram of the essential portions of a second embodiment of the invention which employs modulated pulse light shown at (1) in Figure 7 which is a time chart corresponding to Figure 2. As compared with the first embodiment of the invention shown in Figure 1, the second embodiment of the invention differs only in that a generator 1' for the modulated pulse voltage is employed instead of the generator 1 for the simple pulse voltage, that a bandpass filter 11 which passes only current of a modulation pulse frequency based on a modulated pulse voltage having developed in the photoelectric element 4 for receiving the light, and a half-wave rectifier circuit 12 which rectifies the output of the amplifier 5, are respectively disposed before and after the amplifier 5 for the output voltage of the element 4, and that a peak value holding circuit 6' is employed instead of the peak value holding circuit 6. The same portions on the right side as in Figure 1 have been omitted for want of space. More specifically, in the circuit 6, the output voltage of the amplifier 5 is differentiated through the series circuit consisting of the diode $D_2$, capacitor $C_2$ and resistor $R_2$, and the transistor $T_1$ is turned "on" by the voltage drop having appeared across the resistor $R_2$. In contrast, the circuit 6' includes a waveshaping circuit 13 wherein the output voltage of the half-wave rectifier circuit 12 shown at (2) in Figure 7 is applied through a resistor $R_{18}$ to the noninverting input terminal (+) of a comparator $CM_4$, to the inverting input terminal (−) of which is coupled the intermediate point of resistors $R_{19}$ and $R_{20}$ connected in series to the same power source as that E in the receiver Re shown in Figure 1, and wherein the aforementioned output voltage is waveshaped through the comparator $CM_4$ as shown at $(2_1)$ in Figure 7; and a charge-and-discharge circuit 14 for the output of the circuit 13, wherein as shown at $(2_2)$ in Figure 7, a capacitor $C_7$ is once charged with the output of the circuit 13 through a resistor $R_{21}$ as well as a diode $D_2$, and the charges of the capacitor $C_7$ are discharged through a discharge resistor $R_{22}$ before the circuit 13 provides the next output thereof. The output voltage of the circuit 14, namely, the voltage of the capacitor $C_7$ shown at $(2_2)$ in Figure 7 is differentiated through the series circuit consisting of the capacitor $C_2$ and the resistor $R_2$, as shown at (3) in Figure 7, and the transistor $T_1$ is turned "on" by the voltage drop appearing across the resistor $R_2$. Both the embodiments agree in the point that the conduction of the transistor $T_5$ of the light path interception-discriminating circuit is controlled by the voltage of the input side of the differentiation circuit which has the capacitor $C_2$ and the resistor $R_2$ connected in series.

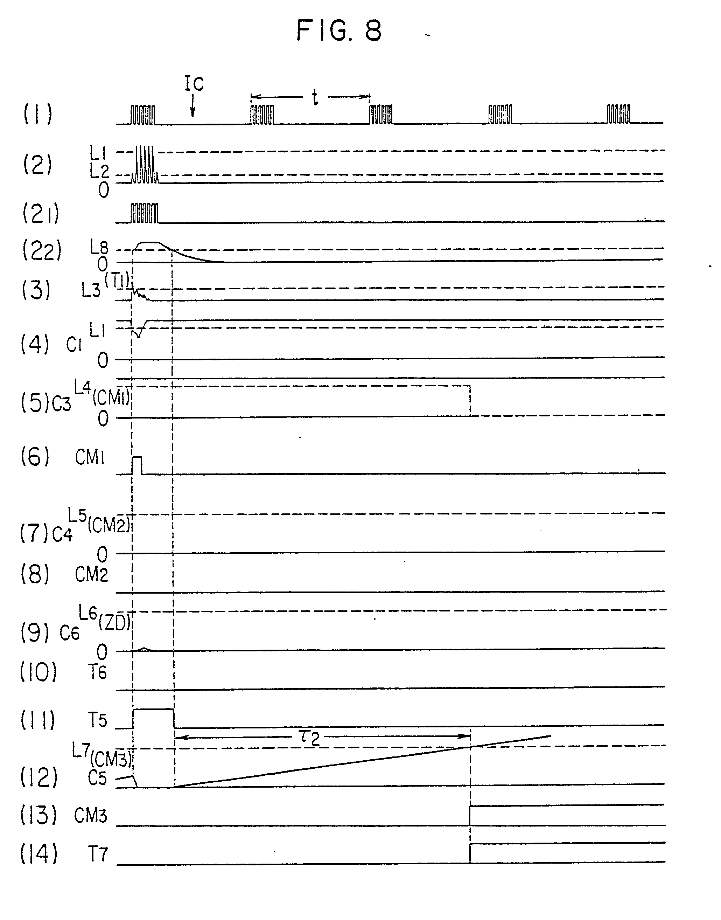

Figures 7 and 8 are time charts corresponding to Figures 2 and 4 and showing the operating states of various parts as to the embodiment shown in Figure 6, in the case where smoke has entered the light path in the normal state at a point of time indicated by arrow Sm and where the smoke density has changed slowly, and in the case where the interception of the light path has occurred in the normal state at a point of time indicated by arrow Ic, respectively. In these time charts, (0) illustrates the density variation of the smoke in the light path, (1) the modulated pulse light emission of the light emitting element 2 having a period $t$, (2) the output voltage obtained from the received light output of the photoelectric element 4 passing through the bandpass filter 11, amplifier 5 and half-wave rectifier circuit 12, $(2_1)$ the output voltage of the waveshaping circuit 13 in the peak value holding circuit 6', $(2_2)$ the output voltage of the charge-and-discharge circuit 14 in the same 6', (3) the voltage appearing across the resistor $R_2$ of the circuit 6' i.e. a result of the differentiation of the output voltage of the circuit 14, (4) the peak voltage of each pulse train of the half-wave rectifier circuit 12, this peak voltage being held in the capacitor $C_1$, (5) the maximum value in the output voltages of the circuit 12, this value being stored in the capacitor $C_3$ of the fire discriminating circuit 7, (6) the output of the comparator $CM_1$, (7) the voltage of the capacitor $C_4$ of the delay circuit 8 shown in Figure 1 and connected on the output side of the comparator $CM_1$, (8) the output of the comparator $CM_2$ of the

same circuit as the circuit 8 shown in Figure 1, (9) the voltage of the capacitor $C_6$ of the delay circuit 10, (10) the operating state of the transistor $T_6$, (11) the operating state of the transistor $T_5$ of the light path interception-discriminating circuit 9, (12) the voltage of the capacitor $C_5$, (13) the output of the comparator $CM_3$, and (14) the operating state of transistor $T_7$. Symbol $L_1$ at (0), (2) and (4) denotes the level which is to be determined a fire, symbol $L_2$ at (2) denotes the level which is to be determined light path interception, the levels indicated by $L_3$–$L_7$ at (3)—(12) are the same as those shown in Figures 2 and 4, and symbol $L_8$ at ($2_2$) denotes the voltage level which is required for the conduction of the transistor $T_5$ of the light path interception-discriminating circuit 9.

In this manner, in the embodiment shown in Figure 6, the modulated pulse light of high frequency is used instead of the simple pulse light, and the output voltage of the light receiving photoelectric element 4 is passed through the bandpass filter 11, amplified by the amplifier 5 and rectified by the half-wave rectifier circuit 12. Therefore, due to inductance, resistance etc. in the filter 11 and the amplifier 5, the amplitude of the rectified output voltage becomes smaller at the end parts thereof as seen at (2) and in Figures 7 and 8, and there is a fear that the smoke detector might operate erroneously, (refer to the specification and drawings of Japanese Utility Model Application No. 56-139190 filed by the same Applicant). For this reason, the output waveform is shaped as shown at ($2_1$) by means of the wave-shaping circuit 13 in the peak value holding circuit 6′. The capacitor $C_7$ of the charge-and-discharge circuit 14 is charged with the shaped output voltage through the resistor $R_{21}$ and the diode $D_2$ as illustrated at ($2_2$). Each time a charging current flows through the capacitor $C_7$, the voltage shown at (3) develops which is the differentiated voltage of the capacitor $C_7$ across the resistor $R_2$ through the capacitor $C_2$. Only when the peak value of the voltage has exceeded the level $L_3$, does the transistor $T_1$ turn "on" to discharge the charge of the capacitor $C_1$ as shown at (4). After the end of the discharge, the capacitor $C_1$ is recharged with the output voltage of the half-wave rectifier circuit 12 shown at (2), until the voltage of this capacitor reaches the peak value of the output voltage shown at (2) of each figure. Except for these points, the operating states do not differ from those of the various portions of the embodiment of Figure 1 as illustrated in Figures 2 and 4. In addition, the operating states of the various portions of the embodiment of Figure 6, corresponding to those in the embodiment of Figure 1, in the case shown in Figure 3 where high density smoke equal to or above the light path interception level has entered the light path at first and where low density smoke equal to or above the fire level has subsequently entered and in the case shown in Figure 5 where the interception of the light path has occurred at a point of time indicated by the arrow Ic during the light emission of the light emitting element 2, can be readily conjectured from the operating states of the various portions shown in Figures 7 and 8 because the parts ($2_2$) of Figures 7 and 8 correspond to the parts (2) of Figures 2—5. Regarding the relationships of length among the periods of time $\tau_1$, $\tau_2$ and $\tau_3$, the embodiment of Figure 6 is the same as that of Figure 1, except in the point that $t_c$ denotes the conduction time of the transistor $T_5$ which is determined by the waveform of the output voltage of the charge-and-discharge circuit 14 shown at ($2_2$), corresponding to the width of the pulse train equal to or above the level $L_2$ of the half-wave rectified output shown at (2) of Figure 7 or Figure 8, instead of the width of the pulse output shown at (2) of each of Figures 2—5.

As set forth above, a light extinction type smoke detector employing pulse light according to each of the two embodiments of the invention produces, owing to a simple and appropriate arrangement, the effect that energy consumption is reduced by employing simple pulse light or modulated pulse light in place of continuous lighting and that smoke due to a fire and the interception of a light path which extends from a light source to a photoelectric element receiving the light of the light source are correctly discriminated and detected, so that a false alarm can be prevented.

**Claims**

1. A light extinction type smoke detector including a pulse light emitting element (2) which emits light pulses through a passageway (3) for the smoke of a fire to a photoelectric element (4), a peak value holding circuit (6) and a fire discriminating circuit (7) responsive to the output of the peak value holding circuit (6), and a light path interception-discriminating circuit (9) characterized in that

the light path interception-discriminating circuit (9) is responsive to the output of the photoelectric element (4) to produce an output only when an interception of the light path within the passageway (3) has occurred and continued for at least a predetermined period of time $\tau_2$;

when the light path interception-discriminating circuit (9) provides an output, the operation of the fire discriminating circuit (7) is immediately inhibited, and a light path interception signal is simultaneously provided;

when the fire discriminating circuit (7) provides an output, the operation of the light path interception-discriminating circuit (9) is inhibited after a delay of a predetermined period of time $\tau_3$, and a fire signal is provided after a delay of a predetermined period $\tau_1$;

the lengths of the predetermined periods $\tau_1$, $\tau_2$, and $\tau_3$ are so related that while the period of time $\tau_3$ is not longer than the time period $\tau_1$ the light path interception signal is set so as to be provided before the provision of the fire signal and the inhibition of the operation of said light path interception-discriminating circuit (9).

2. A light extinction smoke detector as claimed in claim 1 characterized in that

the light pulses emitted by the pulsed light emitting element (2) are modulated, and the peak value holding circuit (6') includes a waveshaping circuit (13) and a charge-and-discharge circuit (14) connected to the output of the waveshaping circuit (13), the input of the light path interception-discriminating circuit (9) being connected to the output of the charge-and-discharge circuit (14).

### Patentansprüche

1. Ein Licht-Extinktions-Rauch-Detektor mit einem pulsierendes Licht aussendenden Element (2), welches Lichtpulse durch einen für das Eindringen von Rauch eines Feuers ausgebildeten Zwischenraum (3) zu einem Photoelement (4) sendet, einem Spitzenwert-Halte-Schaltkreis (6) und einem Brand-UnterscheidungsSchaltkreis, der auf das Ausgangssignal des Spitzenwert-Halte-Schaltkreises (6) anspricht und einem Lichtweg - Unterbrechungs - Unterscheidungs - Schaltkreis (9) dadurch gekennzeichnet, dass der Lichtweg - Unterbrechungs - Unterscheidungs - Schaltkreis (9) auf das Ausgangssignal des Photoelements (4) anspricht und eine Ausgangssignal nur dann abgibt, wenn eine Unterbrechung des Lichtwegs innerhalb des Zwischenraums (3) mindestens für eine Zeitspanne von TAU2 eingetreten ist;

wenn der Lichtweg-Unterbrechungs-Unterscheidungs-Schaltkreis (9) ein Ausgangssignal abgibt, die Betätigung des Brand-Unterscheidungs-Schaltkreises (7) sofort blockiert wird und gleichzeitig ein Lichtweg-Unterbrechungs-Signal abgegeben wird;

wenn der Brand-Unterscheidungs-Schaltkreis (7) ein Ausgangssignal abgibt, die Betätigung des Lichtweg - Unterbrechungs - Unterscheidungs - Schaltkreises (9) nach einer vorbestimmten Verzögerungszeit TAU3 blockiert wird und nach einer vorbestimmten Verzögerungszeit TAU1 ein Brandalarmsignal abgegeben wird, wobei die Längen der vorbestimmten Zeitspannen TAU1, TAU2 und TAU3 so zusammenhängen, dass die Zeitspanne TAU3 nicht länger als die Zeitspanne TAU1 ist, dass das Lichtweg-Unterbrechungs-Signal so gesetzt ist, dass es vor der Abgabe des Brandalarmsignals und der Blockierung der Betätigung des Lichtweg - Unterbrechungs - Unterscheidungs - Schaltkreises (9) abgegeben wird.

2. Ein Licht-Extinktions-Rauch-Detektor gemäss Patentanspruch 1, dadurch gekennzeichnet, dass die von dem Licht emittierenden Element (2) ausgesandten Lichtpulse moduliert sind und der Spitzenwert-Halte-Schaltkreis (6') einen Wellenformbildungsschaltkreis (13) und einen Auflade/ Entlade-Schaltkreis (14), welcher mit dem Ausgang des Wellenformbildungsschaltkreises (13) verbunden ist, aufweist, wobei der Eingang des Lichtweg - Unterbrechungs - Unterscheidungs-Schaltkreises (9) mit dem Ausgang des Auflade/ Entlade-Schaltkreises (14) verbunden ist.

### Revendications

1. Détecteur une fumée du type à extinction de lumière comprenant un élément émettant une impulsion lumineuse (2) qui émet des impulsions lumineuses par un passage (3) pour la fumée d'un feu vers un élément photo-électrique (4), un circuit de maintien de valeur de crête (6) et un circuit de discrimination de feu (7) agissant en réponse à la sortie du circuit de maintien de valeur de crête (6), et un circuit de discrimination d'interception de trajet lumineux (9), caractérisé en ce que:

le circuit (9) de discrimination d'interception de trajet lumineux répond à la sortie de l'élément photo-électrique (4) pour produire une sortie seulement quand une interception du trajet lumineux dans le passage (3) a eu lieu et a continué pendant au moins une durée prédéterminée $Tau_2$;

quand le circuit (9) de discrimination d'interception de trajet lumineux fournit une sortie, le fonctionnement du circuit de discrimination de feu (7) est immédiatement inhibé, et un signal d'interception de trajet lumineux est simultanément fourni;

quand le circuit de discrimination de feu (7) fournit une sortie, le fonctionnement du circuit (9) de discrimination d'interception de trajet lumineux est inhibé après un retard d'une durée $Tau_3$ prédéterminée, et un signal de feu est fourni après un retard d'une durée prédéterminée $Tau_1$;

les longueurs des durées prédéterminées $Tau_1$, $Tau_2$ et $Tau_3$ sont liées de sorte que, quand la durée $Tau_3$ n'est pas supérieure à la durée $Tau_1$, le signal d'interception de trajet lumineux est établi de façon à être fourni avant la fourniture du signal de feu et l'inhibition du fonctionnement du circuit (9) de discrimination d'interception de trajet lumineux.

2. Détecteur de fumée à extinction de lumière selon la revendication 1, caractérisé en ce que: les impulsions lumineuses émises par l'élément émettant de la lumière en impulsions (2) sont modulées et le circuit de maintien de valeur de crête (6') comprend un circuit de mise en forme (13) et un circuit de charge et de décharge (14) connecté à la sortie du circuit de mise en forme (13), l'entrée du circuit (9) de discrimination d'interception de trajet lumineux étant connectée à la sortie du circuit de charge et de décharge (14).

FIG. I

# FIG. 2

FIG. 3

# FIG. 4

# FIG. 5

FIG. 6

FIG. 7

# FIG. 8